# EUROPÄISCHE PATENTSCHRIFT

(11) **EP 2 774 529 B1**
(45) Veröffentlichungstag und Bekanntmachung des Hinweises auf die Patenterteilung: **07.09.2022**
(21) Anmeldenummer: 14157993.8
(22) Anmeldetag: 06.03.2014
(51) Int. Cl.: A61B 1/00, G02B 23/24

(54) **Endoskop mit Bildaufrichtungseinrichtung**
Endoscope with image erection device
Endoscope doté d'un dispositif d'orientation d'image

(30) Priorität: 08.03.2013 DE 102013102343; 09.08.2013 DE 102013108631
(43) Veröffentlichungstag der Anmeldung: 10.09.2014
(73) Patentinhaber: Karl Storz SE & Co. KG, 78532 Tuttlingen (DE)
(72) Erfinder: Weiger, Ulrich, Dr., 78532 Tuttlingen (DE)
(74) Vertreter: Wimmer, Stephan

(56) Entgegenhaltungen:
- DE-T2- 60 015 375
- GB-A- 1 088 431
- US-A1- 2011 199 471

## Beschreibung

Die vorliegende Erfindung ist auf ein Endoskop mit einer Bildaufrichtungseinrichtung zum Aufrichten eines mittels des Endoskops erfassten Bilds, insbesondere auf die Ausgestaltung der Bildaufrichtungseinrichtung, und auf ein Verfahren zum Aufrichten eines Bilds in einem Endoskop bezogen.

In vielen starren Endoskopen werden Relaislinsensysteme aus mehreren hintereinander angeordneten Stablinsen verwendet. Jedes Element des Relaislinsensystems, insbesondere jede Stablinse, bewirkt eine vollständige Umkehr des Bilds, d.h. einen Seitentausch sowohl in vertikaler als auch in horizontaler Richtung, gleichbedeutend mit einer Rotation um 180 Grad um die optische Achse des Relaislinsensystems. Bei geradsichtigen Endoskopen bzw. Endoskopen mit Blickrichtung parallel zur Längsachse des Schafts wird aufgrund einer geeigneten (insbesondere ungeraden) Anzahl von Elementen des Relaislinsensystems bzw. Stablinsen ein aufrechtes und seitenrichtiges Bild im Okular für den Benutzer sichtbar. Dies gilt auch bei nicht geradsichtigen Endoskopen, die zur Umlenkung des von einem Objekt ausgehenden Lichts ein Bauernfeind-Prisma oder eine andere Anordnung einer geraden Anzahl komplanarer reflektierender Flächen aufweisen.

Endoskope mit einstellbarer Blickrichtung weisen am distalen Ende beispielsweise nur eine reflektierende Fläche auf, die zur Einstellung der Blickrichtung schwenkbar ist (vgl. beispielsweise DE 10 2010 010 948 A1). Die einzelne reflektierende Fläche bewirkt einen Seitentausch in einer Richtung parallel zu einer Ebene, die die Flächennormale der reflektierenden Fläche und die optische Achse vor und nach der reflektierenden Fläche enthält.

Alternativ sind beispielsweise zwei reflektierende Flächen am distalen Ende des Endoskops vorgesehen, von denen eine zur Einstellung der Blickrichtung schwenkbar ist (vgl. beispielsweise DE 10 2010 033 425 A1). Beim Schwenken der schwenkbaren reflektierenden Fläche und damit der Blickrichtung rotiert das beobachtete Bild.

Zur Bildaufrichtung bzw. zur Rückrotation des Bilds ist ein Prisma nach Dove/Amici oder einem Prisma nach Abbe/König verwendbar. Beide Prismen weisen spezifische Nachteile auf.

Die DE 600 15 375 T2 ist die Grundlage für die Präambel des unabhängigen Anspruchs 1 und zeigt ein Endoskop mit einem Rohr, einer distalen Sichtöffnung und Einrichtungen zum Empfangen und Übertragen eines Bildes sowie einen in 45°-Winkel angestellten ersten Reflektor, um das Bild um eine Achse senkrecht zur Endoskop-Längsachse zu drehen. Das Endoskop umfasst weiterhin einen ebenso in 45°-Winkel angestellten zweiten Reflektor, so dass das Bild vom ersten Reflektor zum zweiten Reflektor und von diesem in die Bildempfangseinrichtung reflektiert wird. Da hier zwei Reflexionen erfolgen, ist das Bild entsprechend dem betrachteten Objekt ausgerichtet und muss nicht mehr aufgerichtet werden.

Die US 2011/0199471 A1 geht auf eine Endoskopoptik ein, bei der ein erster Reflektor längs einfallendes Licht rechtwinklig ablenkt, eine zweite Reflexionseinrichtung, die mittels drei Reflexionen den Lichtstrahl zusammen um 180° zurück ablenkt und ein dritter Reflektor, der den Lichtstrahl um weitere 90° wieder in die Ebene des einfallenden Lichtstrahls ablenkt. Um das Bild wieder aufzurichten beinhaltet das Endoskop weiterhin ein Bildaufricht-Prisma mit trapezförmigem Querschnitt.

Aus der GB 1,088,431 ist ein Prismenumkehrsystem mit einem Eintrittsprisma mit einer Eintrittsfläche und zwei Reflexionsflächen und einem Austrittsprisma mit Reflexionsfläche und einer Austrittsfläche beschrieben, die parallel zur Eintrittsfläche ist. Bei einigen Ausführungsbeispielen ist ein drittes Prisma zwischen Eintritts- und Austrittsprisma umfasst, wobei das Austrittsprisma in diesem Ausführungsbeispielen zwei Reflexionsflächen aufweist. Es ergibt sich dabei immer eine ungeradezahlige Anzahl an Reflexionen, so dass das Bild verdreht gegenüber dem betrachteten Objekt ist.

Die DE 2347914 stellt ein Endoskop mit einer Fiberoptik einem Prismen-System vor. Dieses besteht in den verschiedenen Ausführungsbeispielen aus zwei Prismen bzw. einem Dachkantprisma. Hier wird zwar mit einem Kompensationssystem die Drehung eines Prismas kompensiert, nicht jedoch ein Bild in Bezug auf Bild - Spiegelbild in die Lage des Objekts aufgerichtet.

Eine Aufgabe der vorliegenden Erfindung besteht darin, ein verbessertes Endoskop mit einer Bildaufrichtungseinrichtung zum Aufrichten eines Bilds in einem Endoskop zu schaffen.

Diese Aufgabe wird durch den Gegenstand des unabhängigen Anspruchs 1 gelöst. Weiterbildungen sind in den abhängigen Ansprüchen angegeben.

Ein Endoskop umfasst eine Bildübertragungseinrichtung in einem Schaft zur Übertragung eines Bild vom distalen Ende zum proximalen Ende des Endoskops und eine Bildaufrichtungseinrichtung zum Aufrichten eines mittels des Endoskops erfassten Bilds, wobei die Bildaufrichtungseinrichtung eine erste winkelabhängig reflektierende Fläche und eine zweite winkelabhängig reflektierende Fläche aufweist, und wobei die Bildaufrichtungseinrichtung so angeordnet und ausgebildet ist, dass Licht zur Bildaufrichtung nacheinander an der ersten reflektierenden Fläche reflektiert wird, durch die erste reflektierende Fläche und durch die zweite, reflektierende Fläche hindurchtritt und an der zweiten reflektierenden Fläche reflektiert wird.

Das Endoskop kann für medizinische oder für nicht-medizinische, insbesondere technische, Anwendungen ausgebildet sein. Das Endoskop weist insbesondere am distalen Ende des Schafts eine reflektierende Fläche zum Einkoppeln von von einem Objekt ausgehendem Licht in die Bildübertragungseinrichtung auf. Die Orientierung der reflektierenden Fläche definiert die Blickrichtung des Endoskops. Insbesondere ist die reflektierende Fläche zur Einstellung der Blickrichtung des Endoskops schwenkbar.

Die Bildübertragungseinrichtung umfasst insbesondere eine Mehrzahl hintereinander angeordneter Stablinsen oder ein anderes Relaislinsensystem. Alternativ oder zusätzlich kann die Bildübertragungseinrichtung - insbesondere wenn oder soweit der Schaft des Endoskops flexibel ist - ein geordnetes Bündel von Lichtleitfasern aufweisen. Die Bildübertragung überträgt das Bild in Form von Licht, das von einem zu beobachtenden Objekt ausgeht.

Die Bildaufrichtungseinrichtung kann distal oder proximal der Bildübertragungseinrichtung oder zwischen zwei Teilen der Bildübertragungseinrichtung (insbesondere zwischen zwei Stablinsen) angeordnet sein. Das durch die Bildaufrichtungseinrichtung bewirkte Aufrichten des mittels des Endoskops erfassten Bilds besteht in einer Seitenumkehr in einer Richtung, die insbesondere in einer Ebene liegt, die die Flächennormale der reflektierenden Fläche (im Falle von deren Krümmung: in deren Mitte) und die optische Achse der Bildübertragungseinrichtung enthält.

Beide winkelabhängig reflektierende Flächen der Bildaufrichtungseinrichtung sind insbesondere jeweils eben. Beide winkelabhängig reflektierende Flächen sind insbesondere dachförmig angeordnet.

Die Ausgestaltung der Bildaufrichtungseinrichtung mit zwei winkelabhängig reflektierenden Flächen und insbesondere die Anordnung und Ausgestaltung der Bildaufrichtungseinrichtung derart, dass das von dem zu beobachtenden Objekt ausgehende Licht nacheinander an der ersten reflektierenden Fläche reflektiert wird, durch die erste reflektierende Fläche und durch die zweite reflektierende Fläche hindurchtritt und an der zweiten reflektierenden Fläche reflektiert wird, kann eine besonders kompakte Ausgestaltung der Bildaufrichtungseinrichtung ermöglichen. Wie insbesondere aus den nachfolgend dargestellten weiteren Eigenschaften und Merkmalen der Bildaufrichtungseinrichtung und aus der Beschreibung der Ausführungsformen hervorgeht, kann die Bildaufrichtungseinrichtung einen Querschnitt (in einer Ebene senkrecht zur optischen Achse der Bildübertragungseinrichtung) aufweisen, der nur wenig größer ist als der Strahlquerschnitt. Die kompakte Ausgestaltung der Bildaufrichtungseinrichtung ist besonders vorteilhaft, wenn die Bildaufrichtungseinrichtung zur Rückrotation eines Bilds, das um einen von der eingestellten Blickrichtung abhängigen Winkel rotiert ist, ihrerseits rotierbar ist. Ferner kann die kompakte Ausgestaltung eine Anordnung der Bildaufrichtungseinrichtung am distalen Ende oder an einem anderen Ort im Schaft des Endoskops ermöglichen.

Bei einem Endoskop, wie es hier beschrieben ist, ist die Bildaufrichtungseinrichtung insbesondere so angeordnet und ausgebildet, dass Licht zur Bildaufrichtung nacheinander an der ersten reflektierenden Fläche reflektiert wird, an einer dritten reflektierenden Fläche reflektiert wird, durch die erste reflektierende Fläche hindurchtritt, an einer vierten reflektierenden Fläche reflektiert wird, durch die zweite reflektierende Fläche hindurchtritt, an einer fünften reflektierenden Fläche reflektiert wird und an der zweiten reflektierenden Fläche reflektiert wird.

Die dritte reflektierende Fläche, die vierte reflektierende Fläche und die fünfte reflektierende Fläche sind jeweils insbesondere winkelunabhängig reflektierend, und dazu jeweils insbesondere verspiegelt. Die dritte reflektierende Fläche, die vierte reflektierende Fläche und die fünfte reflektierende Fläche sind jeweils insbesondere eben.

Bei einem Endoskop, wie es hier beschrieben ist, sind die dritte reflektierende Fläche, die vierte reflektierende Fläche und die fünfte reflektierende Fläche insbesondere parallel zueinander angeordnet.

Bei einem Endoskop, wie es hier beschrieben ist, sind die dritte reflektierende Fläche, die vierte reflektierende Fläche und die fünfte reflektierende Fläche insbesondere parallel zur optischen Achse der Bildübertragungseinrichtung angeordnet.

Bei einem Endoskop, wie es hier beschrieben ist, sind alle reflektierenden Flächen der Bildaufrichtungseinrichtung, die an der Bildaufrichtung beteiligt sind, insbesondere eben und komplanar.

An der Bildaufrichtung beteiligt sind alle reflektierende Flächen, an denen Licht zur Bildaufrichtung reflektiert werden muss, an denen also nicht nur Licht in einem Randbereich des Strahlenbündels und/oder aufgrund von Fertigungstoleranzen oder eines Justagefehlers reflektiert wird. An der Bildaufrichtung beteiligt sind die erste, winkelabhängig reflektierende Fläche, die zweite, winkelabhängig reflektierende Fläche sowie die dritte, vierte und fünfte (jeweils insbesondere winkelunabhängig) reflektierenden Flächen.

Bei einem Endoskop, wie es hier beschrieben ist, weist die Bildaufrichtungseinrichtung im Wesentlichen die Gestalt eines Quaders auf, wobei der Quader in einer Ebene senkrecht zur optischen Achse der Bildübertragungseinrichtung einen im Wesentlichen quadratischen Querschnitt aufweist.

Die Gestalt der Bildaufrichtungseinrichtung kann insbesondere an Kanten von der Gestalt eines idealen Quaders abweichen, indem die Kanten zum Schutz vor einer Beschädigung mit Fasen versehen sind. Ferner weicht die Gestalt der Bildaufrichtungseinrichtung von der Gestalt eines idealen Quaders insbesondere dadurch ab, dass die Bildaufrichtungseinrichtung aus mehreren Prismen zusammengesetzt sein kann, zwischen denen schmale Spalte und/oder Stufen an den Außenflächen vorgesehen sein können. Ferner kann die Gestalt der Bildaufrichtungseinrichtung von der Gestalt eines idealen Quaders dahin gehend abweichen, dass die Lichteintrittsfläche und die Lichtaustrittsfläche der Bildaufrichtungseinrichtung jeweils gekrümmt und/oder durch Stablinsen oder andere Einrichtungen mit nicht-quadratischem Querschnitt verändert oder ergänzt sein können.

Bei einem Endoskop, wie es hier beschrieben ist, sind insbesondere an zwei einander gegenüberliegenden der sechs ebenen Oberflächenabschnitte des Quaders die Lichteintrittsfläche der Bildaufrichtungseinrichtung und die Lichtaustrittsfläche der Bildaufrichtungseinrichtung angeordnet und an zwei weiteren einander gegenüberliegenden der sechs ebenen Oberflächenabschnitte des Quaders einerseits die vierte reflektierende Fläche und andererseits die dritte und die fünfte reflektierende Fläche angeordnet.

Bei einem Endoskop, wie es hier beschrieben ist, umfasst die Bildaufrichtungseinrichtung insbesondere ein erstes Prisma, ein zweites Prisma und ein drittes Prisma, wobei das erste Prisma eine Lichteintrittsfläche der Bildaufrichtungseinrichtung, die erste reflektierende Fläche und die dritte reflektierende Fläche umfasst, wobei das zweite Prisma die vierte reflektierende Fläche, eine Lichteintrittsfläche gegenüber und parallel zu der ersten reflektierenden Fläche und eine Lichtaustrittsfläche gegenüber und parallel zu der zweiten reflektierende Fläche umfasst, und wobei das dritte Prisma eine Lichtaustrittsfläche der Bildaufrichtungseinrichtung, die zweite reflektierende Fläche und die fünfte reflektierende Fläche umfasst.

Insbesondere sind das erste Prisma und das dritte Prisma gleich und symmetrisch zueinander angeordnet. Dabei sind die Lichteintrittsflächen der Bildaufrichtungseinrichtung am ersten Prisma und die Lichtaustrittsfläche der Bildaufrichtungseinrichtung am dritten Prisma gleich groß und insbesondere eben und parallel zueinander. Die dritte reflektierende Fläche am ersten Prisma und die fünfte reflektierende Fläche am dritten Prisma sind insbesondere gleich groß, parallel zueinander und liegen in einer gemeinsamen Ebene. Am ersten Prisma ist die Lichteintrittsfläche insbesondere senkrecht zur dritten reflektierenden Fläche. Am dritten Prisma ist die Lichtaustrittsfläche insbesondere senkrecht zur fünften reflektierenden Fläche. Die erste reflektierende Fläche, die zweite reflektierende Fläche, die Lichteintrittsfläche des zweiten Prismas und die Lichtaustrittsfläche des zweiten Prismas sind insbesondere gleich groß oder im Wesentlichen gleich groß.

Insbesondere sind ein Randbereich der ersten reflektierenden Fläche am ersten Prisma mit einem Randbereich der Lichteintrittsfläche des zweiten Prismas und ein Randbereich der zweiten reflektierenden Fläche am dritten Prisma mit einem Randbereich der Lichtaustrittsfläche des zweiten Prismas verklebt. Dabei definieren je ein (insbesondere blendenförmiger) Abstandshalter vorbestimmte kleine Abstände zwischen der ersten reflektierenden Fläche am ersten Prisma und der Lichteintrittsfläche des zweiten Prismas bzw. zwischen der zweiten reflektierenden Fläche am dritten Prisma und der Lichtaustrittsfläche des zweiten Prismas.

Der beschriebene Aufbau der Bildaufrichtungseinrichtung aus drei Prismen, die insbesondere in Randbereichen einander gegenüberliegender Oberflächenabschnitte miteinander verklebt sind, ermöglicht eine besonders robuste und dauerhafte Justierung der drei Prismen und damit auch aller reflektierender Flächen zueinander.

Bei einem Endoskop, wie es hier beschrieben ist, sind insbesondere die erste reflektierende Fläche und die zweite reflektierende Fläche der Bildaufrichtungseinrichtung jeweils in einem Winkel von 30 Grad zur optischen Achse der Bildübertragungseinrichtung angeordnet.

Bei der Reflexion an der ersten reflektierenden Fläche beträgt der Winkel zwischen dem Licht und der Flächennormale ca. 60 Grad. Nach der Reflexion an der ersten reflektierenden Fläche trifft das Licht in einem Winkel von ca. 30 Grad zur Flächennormalen auf die dritte reflektierende Fläche, wird dort reflektiert, tritt parallel zur Flächennormalen durch die erste reflektierende Fläche hindurch, trifft in einem Winkel von ca. 30 Grad zur Flächennormalen auf die vierte reflektierende Fläche, wird dort reflektiert, tritt parallel zur Flächennormalen durch die zweite reflektierende Fläche hindurch, trifft in einem Winkel von ca. 30 Grad zur Flächennormalen auf die fünfte reflektierende Fläche und wird an dieser reflektiert. Schließlich trifft das Licht in einem Winkel von ca. 60 Grad zur Flächennormalen auf die zweite reflektierende Fläche und wird an dieser reflektiert.

Ein Endoskop, wie es hier beschrieben ist, umfasst insbesondere ferner eine weitere reflektierende Fläche am distalen Ende des Endoskops zum Einkoppeln von Licht, das von einem zu beobachtenden Objekt ausgeht, in die Bildübertragungseinrichtung, wobei die weitere reflektierende Fläche zum Einstellen der Blickrichtung um eine Schwenkachse schwenkbar ist, und eine Einrichtung zum Koppeln der Bildaufrichtungseinrichtung mit der weiteren reflektierenden Fläche derart, dass ein Schwenken der weiteren reflektierenden Fläche um die Schwenkachse mit einer Rotation der Bildaufrichtungseinrichtung um eine Rotationsachse parallel zur optischen Achse der Bildübertragungseinrichtung einhergeht.

Die Orientierung der schwenkbaren weiteren reflektierenden Fläche definiert die Blickrichtung des Endoskops, wobei ein Schwenken der reflektierenden Fläche um einen bestimmten Winkel ein Schwenken der Blickrichtung um den gleichen Winkel, um den doppelten Winkel oder um einen anderen Winkel bewirken kann. Die Schwenkachse der weiteren reflektierenden Fläche ist insbesondere orthogonal zur Längsachse des Schafts des Endoskops. Die Einrichtung zum Koppeln der Bildaufrichtungseinrichtung mit der weiteren reflektierenden Fläche ist insbesondere eine mechanische Einrichtung. Alternativ kann die Einrichtung eine elektronische oder eine andere digitale oder analoge Logik und einen oder mehrere Elektromotoren, Ultraschallmotoren oder andere lineare oder rotatorische Antriebseinrichtungen aufweisen. Die Einrichtung zum Koppeln umfasst insbesondere ein Kurvengetriebe zur Umsetzung einer Rotationsbewegung der Bildaufrichtungseinrichtung in eine Translationsbewegung einer Schub- oder Zugstange oder einer anderen Übertragungseinrichtung zum Übertragen der Translationsbewegung zur schwenkbaren weiteren reflektierenden Fläche am distalen Ende des Endoskops. Das distale Ende der Übertragungseinrichtung ist beispielsweise als Pleuel ausgebildet oder über einen Pleuel mit der schwenkbaren weiteren reflektierenden Fläche gekoppelt.

Bei einem Verfahren zum Aufrichten eines Bilds in einem Endoskop wird von einem zu beobachtenden Objekt ausgehendes Licht an einer ersten winkelabhängig reflektierenden Fläche reflektiert, das Licht durch die erste winkelabhängig reflektierende Fläche transmittiert, das Licht durch eine zweite winkelabhängig reflektierende Fläche transmittiert und das Licht an der zweiten winkelabhängig reflektierenden Fläche reflektiert.

Das Verfahren ist insbesondere mittels eines Endoskops ausführbar, wie es hier beschrieben ist. Das Endoskop kann für medizinische oder nicht-medizinische oder technische Anwendungen vorgesehen und ausgebildet sein. Die Schritte werden insbesondere in der angegebenen Reihenfolge ausgeführt.

Vor und/oder nach den beschriebenen Schritten kann das von dem zu beobachtenden Objekt ausgehende Licht von einem distalen Ende zu einem proximalen Ende des Endoskop übertragen werden, insbesondere mittels eines Relaislinsensystems und/oder eines geordneten Bündels von Lichtleitfasern.

Das von dem zu beobachtenden Objekt ausgehende Licht wird insbesondere nach dem Reflektieren an der ersten reflektierenden Fläche und vor dem Transmittieren durch die erste reflektierende Fläche an einer dritten reflektierenden Fläche reflektiert, nach dem Transmittieren durch die erste reflektierende Fläche und vor dem Transmittieren durch die zweite reflektierende Fläche an einer vierten reflektierenden Fläche reflektiert und nach dem Transmittieren durch die zweite reflektierende Fläche und vor dem Reflektieren an der zweiten reflektierenden Fläche an einer fünften reflektierenden Fläche reflektiert. Die dritte reflektierende Fläche, die vierte reflektierende Fläche und die fünfte reflektierende Fläche sind jeweils insbesondere winkelunabhängig reflektierend.

Bei einem Verfahren, wie es hier beschrieben ist, wird von dem zu beobachtenden Objekt ausgehendes Licht vor allen bislang beschriebenen Schritten insbesondere mittels einer weiteren reflektierenden Fläche am distalen Ende eines Schafts des Endoskops reflektiert, wobei die Orientierung der weiteren reflektierenden Fläche die Blickrichtung des Endoskops definiert.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einer Bildaufrichtungseinrichtung;
- Figur 2: eine schematische Darstellung eines weiteren Endoskops mit einer Bildaufrichtungseinrichtung;
- Figur 3: eine schematische Darstellung eines weiteren Endoskops mit einer Bildaufrichtungseinrichtung;
- Figur 4: eine schematische Darstellung einer Bildaufrichtungseinrichtung;
- Figur 5: eine weitere schematische axonometrische Darstellung der Bildaufrichtungseinrichtung aus Figur 4;
- Figur 6: eine schematische Darstellung von Teilen eines Endoskops;
- Figur 7: eine schematische Schnittdarstellung einer Bildaufrichtungseinrichtung und eines Magnetträgers;
- Figur 8: ein schematisches Flussdiagramm eines Verfahrens zum Aufrichten eines Bilds in einem Endoskop.

### Beschreibung der Ausführungsformen

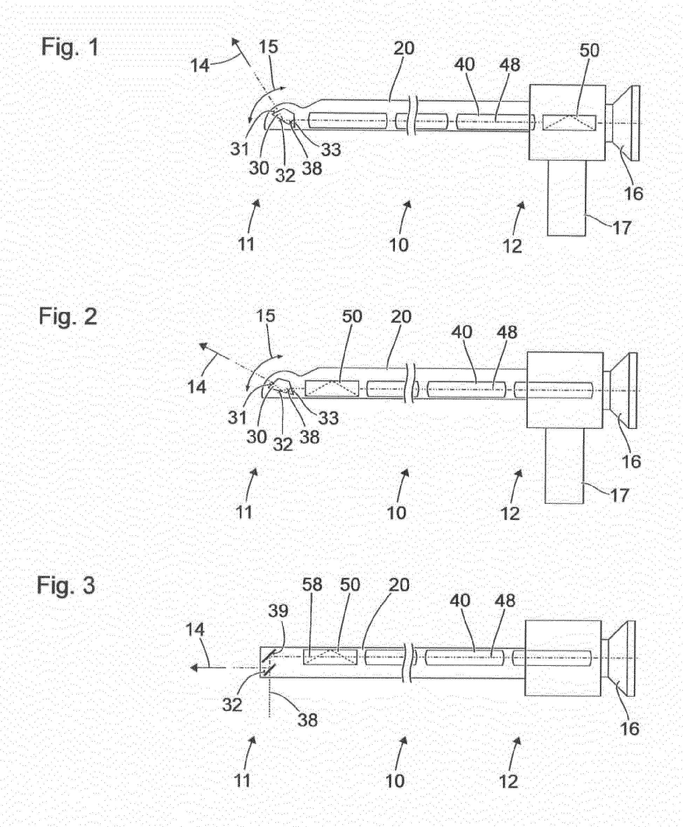

Figur 1 zeigt eine schematische Darstellung eines Endoskops 10 mit einem distalen Ende 11 und einem proximalen Ende 12. Die Blickrichtung 14 des Endoskops 10 ist innerhalb eines durch einen gebogenen Pfeil 15 angedeuteten Bereichs einstellbar. Am proximalen Ende 12 weist das Endoskop 10 ein Okular 16 und eine Kupplung 17 für ein Lichtleitkabel zum Zuführen von Beleuchtungslicht zum Endoskop 10 auf.

Das Endoskop 10 umfasst einen Schaft 20, der sich vom proximalen Ende 12 bis zum distalen Ende 11 des Endoskops erstreckt. Am distalen Ende 11 des Endoskops 10 ist ein Schwenkprisma 30 mit einer distalen Lichteintrittsfläche 31, einer reflektierenden Fläche 32 und einer proximalen Lichtaustrittsfläche 33 angeordnet. Das Schwenkprisma 30 ist insbesondere als Prisma nach Dove/Amici ausgebildet und um eine Schwenkachse 38 senkrecht zur Längsachse des Schafts 20 und senkrecht zur Zeichenebene der Figur 1 schwenkbar. Ein Schwenken des Schwenkprismas 30 um einen Winkel bewirkt ein Schwenken der Blickrichtung 14 um den doppelten Winkel.

Im Schaft 20 ist eine Bildübertragungseinrichtung 40 mit einer optischen Achse 48 angeordnet. Die strichpunktierte Linie, die die optische Achse 48 der Bildübertragungseinrichtung 40 andeutet, ist distal bis zum Schwenkprisma 30 und darüber hinaus in Blickrichtung 14 verlängert, um die Übertragung von Licht, das aus der Blickrichtung 14 einfällt, im Schwenkprisma 30 und in der Bildübertragungseinrichtung 40 anzudeuten. Die Bildübertragungseinrichtung 40 ist insbesondere als Anordnung von Stablinsen, deren Konturen in Figur 1 angedeutet sind oder als ein anderes Relaislinsensystem ausgebildet. Alternativ kann die Bildübertragungseinrichtung 40 ein geordnetes Bündel von Lichtleitfasern aufweisen. In diesem Fall kann der Schaft 20 flexibel ausgebildet sein. Alternativ können ein geordnetes Bündel von Lichtleitfasern und ein Relaislinsensystem hintereinander angeordnet sein.

Am proximalen Ende 12 des Endoskops 10 ist eine Bildaufrichtungseinrichtung 50 zwischen dem proximalen Ende der Bildübertragungseinrichtung 40 und dem Okular 16 angeordnet. Während jedes Relaylinsensystem/Umkehrsystem eine vollständige Bildumkehr bewirkt, bewirken sowohl die reflektierende Fläche 32 am Schwenkprisma 30 als auch die Bildaufrichtungseinrichtung 50 jeweils einen Seitentausch in einer Richtung in der Zeichenebene der Figur 1. Dadurch hebt die Bildaufrichtungseinrichtung 50 die das Bild stürzende Wirkung der reflektierenden Fläche 32 des Schwenkprismas auf und ermöglicht die Beobachtung eines aufrechtenden Bilds durch das Okular 16.

Das Endoskop 10 ist ausgebildet, um das Schwenkprisma 30, die Bildübertragungseinrichtung 40 und die Bildaufrichtungseinrichtung 50 hermetisch dicht zu umschließen, um das Eindringen von Wasserdampf und anderen störend oder zerstörend wirkenden Fluiden und Festkörpern in das optische System zu vermeiden. Dazu weist das Endoskop 10 insbesondere ein gekrümmtes transparentes Fensterbauteil am distalen Ende 11 und ein weiteres transparentes Fensterbauteil am proximalen Ende 12 auf, die in Figur 1 nicht dargestellt sind.

Figur 2 zeigt eine schematische Darstellung eines weiteren Endoskops 10, das in einigen Merkmalen und Eigenschaften dem oben anhand der Figur 1 dargestellten Endoskop ähnelt. Die Zeichenebene der Figur 2 entspricht der Zeichenebene der Figur 1. Nachfolgend sind lediglich Merkmale und Eigenschaften beschrieben, in denen sich das Endoskop 10 aus Figur 2 von dem oben anhand der Figur 1 dargestellten Endoskop unterscheidet.

Das in Figur 2 dargestellte Endoskop 10 weist ebenso wie das oben anhand der Figur 1 dargestellte Endoskop ein Schwenkprisma 30 am distalen Ende auf, das um eine Schwenkachse 38 senkrecht zur Zeichenebene der Figur 2 schwenkbar ist. Um die Wirkung der Orientierung des Schwenkprisma 30 auf die Blickrichtung 14 anzudeuten, ist das Endoskop 10 in Figur 2 mit einer anderen Blickrichtung 14 dargestellt als das Endoskop in Figur 1.

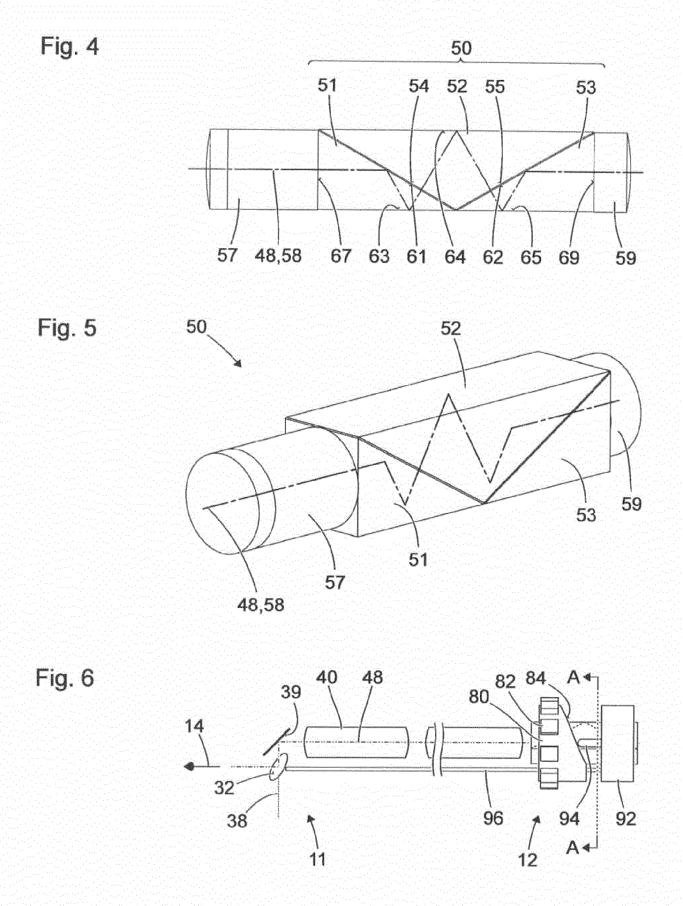

Das Endoskop 10 unterscheidet sich von dem oben anhand der Figur 1 dargestellten Endoskop insbesondere dadurch, dass die Bildaufrichtungseinrichtung 50 nicht am proximalen Ende 12, sondern nahe dem distalen Ende 11 des Endoskops zwischen dem Schwenkprisma 30 und der Bildübertragungseinrichtung 40 angeordnet ist. Die nachfolgend anhand der Figuren 4 und 5 dargestellte kompakte Bauart der Bildaufrichtungseinrichtung 50 kann diese distale Anordnung der Bildaufrichtungseinrichtung 50 ermöglichen ohne einen deutlich vergrößerten Querschnitt des Schafts 20 zu erfordern.

Figur 3 zeigt eine schematische Darstellung eines weiteren Endoskops 10, das in einigen Merkmalen und Eigenschaften dem oben anhand der Figur 1 dargestellten Endoskop und insbesondere dem oben anhand der Figur 2 dargestellten Endoskop ähnelt. Nachfolgend sind lediglich Merkmale und Eigenschaften des Endoskops 10 beschrieben, in denen sich dieses vom oben anhand der Figur 2 dargestellten Endoskop unterscheidet. Die Zeichenebene der Figur 3 enthält zwar ebenso wie die Zeichenebenen der Figuren 1 und 2 die optische Achse 48 der Bildübertragungseinrichtung 40, ist jedoch senkrecht zu der Ebene, in der die einstellbaren Blickrichtungen 14 liegen und damit auch senkrecht zu den Zeichenebenen der Figuren 1 und 2.

Das Endoskop 10 aus Figur 3 unterscheidet sich von dem oben anhand der Figur 2 dargestellten Endoskop insbesondere dadurch, dass am distalen Ende 11 des Endoskops 10 eine schwenkbare reflektierende Fläche 32 und eine feststehende reflektierende Fläche 39 vorgesehen sind. Beide reflektierende Flächen 32, 39 am distalen Ende 11 des Endoskops 10 sind angeordnet und ausgebildet, um Licht jeweils um einen Winkel von 90 Grad umzulenken bzw. abzulenken. Die schwenkbare reflektierende Fläche 32 ist um eine Schwenkachse 38 schwenkbar, die in der Zeichenebene der Figur 3 liegt und senkrecht zur optischen Achse 48 der Bildübertragungseinrichtung 40 und senkrecht zur Längsachse des Schafts 20 ist. Die reflektierende Fläche 39 ist feststehend angeordnet.

Die Flächennormale der schwenkbaren reflektierenden Fläche 32 schließt sowohl mit der Blickrichtung 14 als auch mit der Schwenkachse 38 jeweils einen Winkel von 45 Grad ein. Die Flächennormale der feststehenden reflektierenden Fläche 39 schließt mit der Schwenkachse 38 und mit der Längsachse 48 der Bildübertragungseinrichtung 40 jeweils einen Winkel von 45 Grad ein. Ein Schwenken der schwenkbaren reflektierenden Fläche 32 um einen Winkel bewirkt ein Schwenken der Blickrichtung 14 um den gleichen Winkel. Abweichend von der schematischen Darstellung in Figur 3 können sowohl die schwenkbare reflektierende Fläche 32 als auch die feststehende reflektierende Fläche 39 am distalen Ende 11 des Endoskops 10 jeweils an einem Prisma oder einem ähnlichen transparenten Körper ausgebildet sein, innerhalb dessen jeweils sowohl das zu reflektierende als auch das reflektierte Licht verläuft.

Ein Schwenken der schwenkbaren reflektierenden Fläche 32 am distalen Ende 11 des Endoskops 10 um deren Schwenkachse 38 bewirkt eine Rotation des erfassten Bilds um die optische Achse 48 der Bildübertragungseinrichtung 40. Eine gleichzeitige Rotation der Bildaufrichtungseinrichtung 50 um ihre Rotationsachse 58, die der optischen Achse 48 der Bildübertragungseinrichtung 40 entspricht, macht diese Rotation rückgängig bzw. kompensiert sie.

Figur 4 zeigt eine schematische Darstellung einer Ausführungsform einer Bildaufrichtungseinrichtung 50, die in den oben anhand der Figuren 1 bis 3 dargestellten Endoskopen einsetzbar ist. Die Zeichenebene der Figur 4 entspricht den Zeichenebenen der Figuren 1 bis 3 und enthält die optische Achse 48 der Bildübertragungseinrichtung 40 (vgl. Figuren 1 bis 3) und die Rotationsachse 58 der Bildaufrichtungseinrichtung 50. Figur 5 zeigt eine schematische axonometrische Darstellung der Bildaufrichtungseinrichtung 50. Die Bildaufrichtungseinrichtung 50 ist aus drei Prismen 51, 52, 53 zusammengesetzt. Das erste Prisma 51 und das dritte Prisma 53 sind gleich und spiegelsymmetrisch angeordnet. Das zweite Prisma 52 ist symmetrisch dachförmig und zwischen dem ersten Prisma 51 und dem dritten Prisma 53 angeordnet. Das erste Prisma 51, das zweite Prisma 52 und das dritte Prisma 53 zusammen bilden einen im Wesentlichen quaderförmigen Körper.

Das erste Prisma 51 weist eine erste, winkelabhängig reflektierende Fläche 61 auf, die gegenüber einer Lichteintrittsfläche 54 des zweiten Prismas 52 und parallel zu dieser angeordnet ist. Das dritte Prisma 53 weist eine zweite, winkelabhängig reflektierende Fläche 62 auf, die gegenüber einer Lichtaustrittsfläche 55 des zweiten Prismas 52 und parallel zu dieser angeordnet ist. Das erste Prisma 51 und das zweite Prisma 52 sowie das zweite Prisma 52 und das dritte Prisma 53 sind jeweils starr miteinander verbunden, insbesondere durch Klebungen in einem oder mehreren Randbereichen der ersten reflektierenden Fläche 61 am ersten Prisma 51 und der Lichteintrittsfläche 54 des zweiten Prismas 52 und in einem oder mehreren Randbereichen der zweiten reflektierenden Fläche 62 am dritten Prisma 53 und der Lichtaustrittsfläche 55 des zweiten Prismas 52. Zwischen der ersten reflektierenden Fläche 61 am ersten Prisma 51 und der Lichteintrittsfläche 54 des zweiten Prismas 52 sowie zwischen der zweiten reflektierenden Fläche 62 am dritten Prisma 53 und der Lichtaustrittsfläche 55 des zweiten Prismas 52 sind jeweils dünne Abstandshalter, beispielsweise in Gestalt rahmenförmiger Bauteile, vorgesehen, die in den Figuren 4 und 5 nicht dargestellt sind und vorbestimmte Abstände der einander gegenüberliegenden Fläche gewährleisten.

Am ersten Prisma 51 ist eine dritte, winkelunabhängig reflektierende Fläche 63 vorgesehen. Am zweiten Prisma 52 ist eine vierte, winkelunabhängig reflektierende Fläche 64 vorgesehen. Am dritten Prisma 53 ist eine fünfte, winkelunabhängig reflektierende Fläche 65 vorgesehen. Die dritte reflektierende Fläche 63, die vierte reflektierende Fläche 64 und die fünfte reflektierende Fläche 65 sind jeweils verspiegelt und parallel zueinander und zur optischen Achse 48 der Bildübertragungseinrichtung 40 (vgl. Figuren 1 bis 3) angeordnet. Ferner sind eine Lichteintrittsfläche 67 der Bildaufrichtungseinrichtung 50 am ersten Prisma 51 und eine Lichtaustrittsfläche 69 der Bildaufrichtungseinrichtung 50 am dritten Prisma 53 vorgesehen. Die Lichteintrittsfläche 67 und die Lichtaustrittsfläche 69 der Bildaufrichtungseinrichtung 50 sind jeweils insbesondere eben und parallel zueinander sowie senkrecht zur optischen Achse 48 angeordnet.

Bei dem dargestellten Ausführungsbeispiel sind an der Lichteintrittsfläche 67 und an der Lichtaustrittsfläche 69 der Bildaufrichtungseinrichtung 50 je ein Kreiszylinder 57, 59 aus Glas oder einem anderen transparenten Material angesetzt. Der Kreiszylinder 57 kann mit dem ersten Prisma 51 einstückig gefertigt oder beispielsweise durch Kleben gefügt sein. Der Kreiszylinder 59 kann mit dem dritten Prisma 53 einstückig gefertigt oder beispielsweise durch Kleben gefügt sein. Die Kreiszylinder 57, 59 können jeweils Bestandteil der Bildaufrichtungseinrichtung 50 sein, wobei die Gestalt der Bildaufrichtungseinrichtung 50 zumindest im Bereich der Kreiszylinder 57, 59 von der Gestalt eines Quaders abweicht. Von der Bildaufrichtungseinrichtung 50 abgewandte Stirnflächen der Kreiszylinder 57, 59 sind gekrümmt, so dass die Bildaufrichtungseinrichtung 50 zusammen mit den Kreiszylindern 57, 59 wie eine Stablinse wirkt, die jedoch keine vollständige Bildumkehr, sondern lediglich einen Seitentausch in einer Richtung senkrecht zur Zeichenebene der Figur 4 bewirkt.

Die Bildaufrichtungseinrichtung 50 beispielsweise von links nach rechts durchlaufendes Licht fällt zunächst auf die erste, winkelabhängig reflektierende Fläche 61 am ersten Prisma 51 und wird dort aufgrund des großen Winkels zur Flächennormalen (ca. 60 Grad) totalreflektiert. Danach wird das Licht an der dritten, winkelunabhängig reflektierenden Fläche 63 am ersten Prisma 51 reflektiert, durchtritt im Wesentlichen senkrecht die winkelabhängig reflektierende Fläche 61 am ersten Prisma und die Lichteintrittsfläche 54 am zweiten Prisma 52, wird an der vierten, winkelunabhängig reflektierenden Fläche 64 am zweiten Prisma 52 erneut reflektiert, tritt im Wesentlichen senkrecht durch die Lichtaustrittsfläche 55 des zweiten Prismas 52 und die zweite, winkelabhängig reflektierende Fläche 62 am dritten Prisma 53 und wird an der fünften, winkelunabhängig reflektierenden Fläche 65 reflektiert. Schließlich trifft das Licht flach (mit einem Winkel zur Flächennormalen von ca. 60 Grad) auf die zweite, winkelabhängig reflektierende Fläche 62 und wird dort aufgrund des großen Winkels zur Flächennormalen totalreflektiert, um die Bildaufrichtungseinrichtung 50 nach rechts zu verlassen.

Bei der Bildaufrichtungseinrichtung 50 sind alle an der Bildaufrichtung beteiligten Flächen senkrecht zur Zeichenebene der Figur 4. Die zur Zeichenebene der Figur 4 parallelen Oberflächenabschnitte der Prismen 51, 52, 53 sind an der Bildaufrichtung nicht beteiligt.

Figur 6 zeigt eine schematische Darstellung von Teilen eines Endoskops 10, das in einigen Merkmalen und Eigenschaften dem oben anhand der Figur 3 dargestellten Endoskop ähnelt. Nachfolgend sind lediglich Merkmale und Eigenschaften beschrieben, in denen sich das Endoskop 10 von dem oben anhand der Figur 3 dargestellten Endoskop unterscheidet.

Die Zeichenebene der Figur 6 entspricht der Zeichenebene der Figur 3. Im Unterschied zur Figur 3 sind jedoch keine äußeren Strukturen des Endoskops, insbesondere kein Schaft und keine Bestandteile einer hermetisch dichten Hülle um die optischen Einrichtungen, und nur ein Teil der optischen Einrichtungen dargestellt. Ebenso wie bei den Darstellungen in den Figuren 1 bis 3 ist auch in Figur 6 der Einfachheit halber kein Objektiv am distalen Ende 11 des Endoskops 10 gezeigt, die Bildübertragungseinrichtung 40 ist beispielhaft durch mehrere Stablinsen angedeutet.

Ähnlich wie bei den oben anhand der Figur 3 dargestellten Endoskop weist das in Figur 6 gezeigte Endoskop 10 eine feststehende reflektierende Fläche 39 und eine um eine Schwenkachse 38 in der Zeichenebene der Figur 6 schwenkbare reflektierende Fläche 32 am distalen Ende 11 auf. In Figur 6 ist die schwenkbar reflektierende Fläche 32 gegenüber der in Figur 3 dargestellten Konfiguration bzw. Situation um einen Winkel von ca. 20 bis 30 Grad um die Schwenkachse 38 geschwenkt, so dass die Blickrichtung 14 nicht in der Zeichenebene der Figur 6 liegt, sondern mit dieser einen entsprechenden Winkel einschließt.

Das Endoskop 10 unterscheidet sich von dem oben anhand der Figur 3 dargestellten Endoskop insbesondere dadurch, dass die Bildaufrichtungseinrichtung 50 am proximalen Ende 12 des Endoskops 10 angeordnet ist. Die Bildaufrichtungseinrichtung 50 entspricht der oben anhand der Figuren 4 und 5 dargestellten Bildaufrichtungseinrichtung, wobei Glaszylinder 57, 59 (vgl. Figuren 4, 5) an der Lichteintrittsfläche 67 und an der Lichtaustrittsfläche 69 der Bildaufrichtungseinrichtung 50 in Figur 6 nicht dargestellt oder bei dem anhand der Figur 6 dargestellten Endoskop 10 nicht vorgesehen sind.

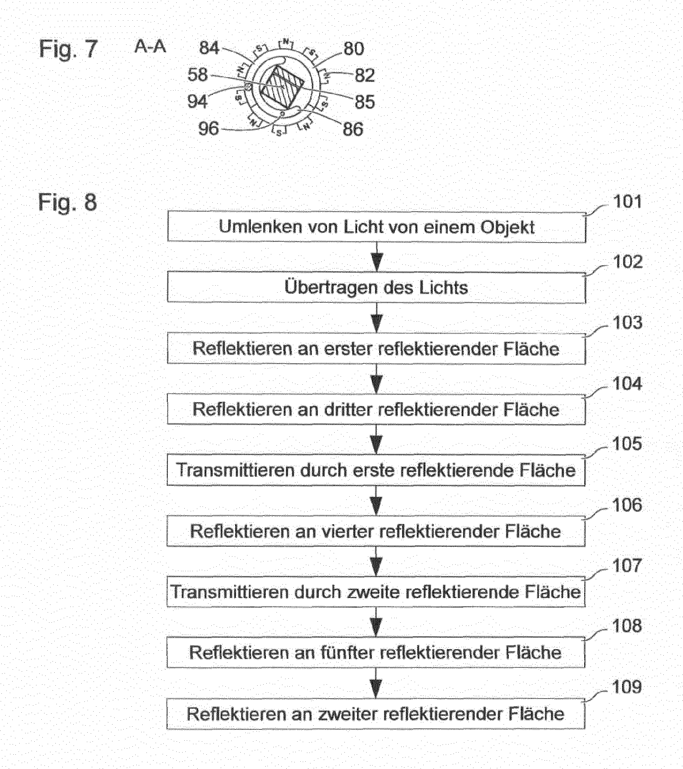

Die Bildaufrichtungseinrichtung 50 ist in einem näherungsweise ringförmigen Magnetträger 80 angeordnet und gehalten, der auch in Figur 7 gezeigt ist. Figur 7 zeigt eine schematische Schnittdarstellung der Bildaufrichtungseinrichtung 50 und des Magnetträgers 80. Die Schnittebene A-A der Figur 7 ist senkrecht zur Zeichenebene der Figur 6 zur optischen Achse 48 der Bildübertragungseinrichtung 40 (vgl. Figur 6) und zur Rotationsachse 58 der Bildaufrichtungseinrichtung 50. Die Lage der Schnittebene A-A ist in Figur 6 angedeutet. Nachfolgend wird auf die Figuren 6 und 7 Bezug genommen.

Der näherungsweise ringförmige Magnetträger 80 weist an seinem äußeren Umfang eine Mehrzahl regelmäßig angeordneter Magnete 82 auf, deren radial äußere Oberflächen alternierend Nord- und Südpole sind. Alle in den Figuren 6 und 7 dargestellten Einrichtungen sind innerhalb einer in den Figuren 6 und 7 nicht dargestellten hermetisch dichten Hülle angeordnet, die zumindest im Bereich des Magnetträgers 80 aus Edelstahl oder einem anderen nicht-ferromagnetischen Material besteht. Außerhalb der nicht dargestellten hermetisch dichten Hülle ist ein korrespondierender Magnetträger mit korrespondierenden Magneten vorgesehen, der manuell gedreht werden kann. Durch die Kopplung der Magnete am in den Figuren 6 und 7 nicht dargestellten manuell drehbaren Magnetträger und der Magneten 82 am in den Figuren 6 und 7 dargestellten Magnetträger 80 wird eine manuell erzeugte Rotationsbewegung auf den Magnetträger 80 übertragen. Die Bildaufrichtungseinrichtung 50 ist in der Mitte des Magnetträgers 80 durch einen Fassungsbereich 85 formschlüssig und kraft- und/oder stoffschlüssig gehalten und mit dem Magnetträger 80 verbunden, so dass jede Rotation des Magnetträgers 80 mit einer entsprechenden Rotation der Bildaufrichtungseinrichtung 50 um die Rotationsachse 58 (die der optischen Achse 48 der Bildübertragungseinrichtung 40 entspricht) einhergeht.

Der in der Ansicht der Figur 7 ringförmige distale Rand des Magnetträgers 80 ist, wie in Figur 6 erkennbar ist, als abschnittsweise rampenförmige Gleitfläche 84 ausgebildet. An einem Schieber 92 ist ein Taststift 94 vorgesehen. Ferner ist der Schieber 92 mit dem proximalen Ende einer Übertragungsstange 96 verbunden, deren distales Ende derart mit der schwenkbaren reflektierenden Fläche 32 mechanisch gekoppelt ist, dass eine Translationsbewegung der Übertragungsstange 96 in Richtung parallel zur Längsachse des Schafts 20 (vgl. Figuren 1 bis 3) bzw. zur optischen Achse 48 der Bildübertragungseinrichtung 40 mit einer Schwenkbewegung der schwenkbaren reflektierenden Fläche 32 um deren Schwenkachse 38 einhergeht. Einzelheiten der mechanischen Kopplung zwischen Übertragungsstange 96 und schwenkbarer reflektierender Fläche 32 sind in Figur 6 nicht dargestellt.

Der Schieber 92, der Taststift 94 und die Übertragungsstange 96 sind durch in den Figuren 6 und 7 nicht dargestellte Einrichtungen so geführt, dass sie lediglich Translationsbewegungen parallel zur optischen Achse 48 der Bildübertragungseinrichtung 40 und zur Rotationsachse 58 der Bildaufrichtungseinrichtung 50 und des Magnetträgers 80 ausführen können. Durch eine in den Figuren 6 und 7 nicht dargestellte Feder oder ein anderes elastisches Element wird der Schieber 92 mit dem Taststift 94 und der Übertragungsstange 96 so weit nach distal (in Figur 6: links) geschoben, dass das distale Ende des Taststifts 94 an der Gleitfläche 84 an der proximalen Seite des Magnetträgers 80 anliegt. Die Übertragungsstange 96 greift durch einen bogenförmigen Schlitz 86 im Magnetträger 80 hindurch.

Aufgrund der rampenförmigen Ausgestaltung der Gleitfläche 84 am Magnetträger 80 geht eine Rotation des Magnetträgers 80 mit der Bildaufrichtungseinrichtung 50 um die Achse 58 mit einer Translationsbewegung des Taststifts 94, des Schiebers 92 und der Übertragungsstange 96 und somit auch mit einer Schwenkbewegung der schwenkbaren reflektierenden Fläche 32 am distalen Ende 11 des Endoskops 10 um deren Schwenkachse 38 einher. Die rampenförmige Gleitfläche 84 und der Taststift 94 bilden ein Kurvengetriebe, das so ausgestaltet ist, dass die beim Schwenken der schwenkbaren reflektierenden Fläche 32 um deren Schwenkachse 38 erzeugte Rotation des erfassten Bilds durch die Rotation der Bildaufrichtungseinrichtung 50 jederzeit rückgängig gemacht bzw. kompensiert wird.

Figur 8 zeigt ein schematisches Flussdiagramm eines Verfahrens zum Aufrichten eines Bilds in einem Endoskop. Obwohl das Verfahren auch mit einem Endoskop ausführbar ist, das Merkmale und Eigenschaften aufweist, die von den oben anhand der Figuren 1 bis 7 dargestellten abweichen, werden nachfolgend beispielhaft Bezugszeichen aus den Figuren 1 bis 7 verwendet, um das Verständnis zu vereinfachen.

Bei einem ersten Schritt 101 wird von einem Objekt ausgehendes (emittiertes, remittiertes oder reflektiertes) und durch ein Fenster am distalen Ende 11 des Endoskops 10 eintretendes Licht abgelenkt bzw. umgelenkt. Wenn das Objekt, von dem das Licht ausgeht, vom distalen Ende 11 des Endoskops 10 aus gesehen in Blickrichtung 14 liegt, die durch die Orientierung der reflektierenden Fläche 32 definiert ist, wird das vom Objekt ausgehende Licht in eine Bildübertragungseinrichtung 40 eingekoppelt.

Bei einem zweiten Schritt 102 wird das beim ersten Schritt 101 umgelenkte und in die Bildübertragungseinrichtung 40 eingekoppelte Licht mittels der Bildübertragungseinrichtung 40 (insbesondere eines Stablinsensystems, eines anderen Relaislinsensystems und/oder eines geordneten Bündels von Lichtleitfasern) vom distalen Ende 11 zum proximalen Ende 12 des Endoskops 10 übertragen.

Bei einem dritten Schritt 103 wird das Licht an einer ersten, winkelabhängig reflektierenden Fläche 61 reflektiert. Bei einem vierten Schritt 104 wird das Licht an einer dritten, winkelunabhängig reflektierenden Fläche 63 reflektiert. Bei einem fünften Schritt 105 wird das Licht durch die erste, winkelabhängig reflektierende Fläche 61 transmittiert bzw. tritt durch die erste, winkelabhängig reflektierende Fläche 61 hindurch. Bei einem sechsten Schritt 106 wird das Licht an einer vierten, winkelunabhängig reflektierenden Fläche 64 reflektiert. Bei einem siebten Schritt 107 wird das Licht durch eine zweite, winkelabhängig reflektierende Fläche 62 transmittiert bzw. tritt durch die zweite, winkelabhängig reflektierende Fläche 62 hindurch. Bei einem achten Schritt 108 wird das Licht an einer fünften, winkelunabhängig reflektierenden Fläche 65 reflektiert. Bei einem neunten Schritt 109 wird das Licht an der zweiten, winkelabhängig reflektierenden Fläche 62 reflektiert.

Der dritte Schritt 103, der vierte Schritt 104, der fünfte Schritt 105, der sechste Schritt 106, der siebte Schritt 107, der achte Schritt 108 und der neunte Schritt 109 werden in der hier beschriebenen Reihenfolge ausgeführt. Der zweite Schritt 102 kann vor dem dritten Schritt 103 oder nach dem neunten Schritt 109 ausgeführt werden.

### Bezugszeichen

- 10: Endoskop
- 11: distales Ende des Schafts 20
- 12: proximales Ende des Schafts 20
- 14: Blickrichtung des Endoskops 10
- 15: Einstellbarkeit der Blickrichtung 12 des Endoskops 10
- 16: Okular des Endoskops 10
- 17: Kupplung für Lichtleitkabel am Endoskop 10
- 20: Schaft des Endoskops 10
- 30: Schwenkprisma am distalen Ende 11 des Endoskops 10
- 31: Lichteintrittsfläche des Schwenkprismas 30
- 32: schwenkbare reflektierende Fläche am distalen Ende 11 des Endoskops 10
- 33: Lichtaustrittsfläche des Schwenkprismas 30
- 38: Schwenkachse des Schwenkprismas 30
- 39: feststehende reflektierende Fläche am distalen Ende 11 des Endoskops 10
- 40: Bildübertragungseinrichtung im Schaft 20
- 48: optische Achse der Bildübertragungseinrichtung 40
- 50: Bildaufrichtungseinrichtung
- 51: erstes Prisma der Bildaufrichtungseinrichtung 50
- 52: zweites Prisma der Bildaufrichtungseinrichtung 50
- 53: drittes Prisma der Bildaufrichtungseinrichtung 50
- 54: Lichteintrittsfläche des zweiten Prismas 52
- 55: Lichtaustrittsfläche des zweiten Prismas 52
- 57: Kreiszylinder
- 58: Rotationsachse der Bildaufrichtungseinrichtung 50
- 59: Kreiszylinder
- 61: erste, winkelabhängig reflektierende Fläche der Bildaufrichtungseinrichtung 50
- 62: zweite, winkelabhängig reflektierende Fläche der Bildaufrichtungseinrichtung 50
- 63: dritte reflektierende Fläche der Bildaufrichtungseinrichtung 50
- 64: vierte reflektierende Fläche der Bildaufrichtungseinrichtung 50
- 65: fünfte reflektierende Fläche der Bildaufrichtungseinrichtung 50
- 67: Lichteintrittsfläche der Bildaufrichtungseinrichtung 50
- 69: Lichtaustrittsfläche der Bildaufrichtungseinrichtung 50
- 80: Magnetträger
- 82: Magnete am Magnetträger 80
- 84: Gleitfläche am Magnetträger 80
- 85: Fassungsbereich für Bildaufrichtungseinrichtung 50 am Magnetträger 80
- 86: Schlitz für Übertragungsstange 96
- 92: Schieber
- 94: Taststift am Schieber 92
- 96: Übertragungsstange zur Übertragung einer Translationsbewegung
- 101: erster Schritt (Umlenken von Licht von einem Objekt)
- 102: zweiter Schritt (Übertragen des Lichts)
- 103: dritter Schritt (Reflektieren des Lichts an einer ersten reflektierenden Fläche)
- 104: vierter Schritt (Reflektieren des Lichts an einer dritten reflektierenden Fläche)
- 105: fünfter Schritt (Transmittieren des Lichts durch die erste reflektierende Fläche)
- 106: sechster Schritt (Reflektieren des Lichts an einer vierten reflektierenden Fläche)
- 107: siebter Schritt (Transmittieren des Lichts durch eine zweite reflektierende Fläche)
- 108: achter Schritt (Reflektieren des Lichts an einer fünften reflektierenden Fläche)
- 109: neunter Schritt (Reflektieren des Lichts an der zweiten reflektierenden Fläche)

## Patentansprüche

1. **Endoskop** (10), mit:
einer **Bildübertragungseinrichtung** (40) in einem Schaft (20) zur Übertragung eines Bilds vom distalen Ende (11) zum proximalen Ende (12) des Endoskops (10);
einer **Bildaufrichtungseinrichtung** (50) zum Aufrichten eines mittels des Endoskops (10) erfassten Bilds,
wobei die Bildaufrichtungseinrichtung (50) eine erste **winkelabhängig reflektierende Fläche** (61) und eine zweite **winkelabhängig reflektierende Fläche** (62) aufweist,
wobei die Bildaufrichtungseinrichtung (50) so angeordnet und ausgebildet ist, dass Licht zur Bildaufrichtung nacheinander an der ersten reflektierenden Fläche (61) reflektiert wird, durch die erste reflektierende Fläche (61) und durch die zweite reflektierende Fläche (62) hindurchtritt und an der zweiten reflektierenden Fläche (62) reflektiert wird,
**dadurch gekennzeichnet, dass**
die Bildaufrichtungseinrichtung (50) im Wesentlichen die Gestalt eines **Quaders** aufweist, wobei der Quader in einer Ebene senkrecht zur optischen Achse der Bildübertragungseinrichtung (40) einen im Wesentlichen quadratischen Querschnitt aufweist.

2. Endoskop (10) nach dem vorangehenden Anspruch, bei dem die Bildaufrichtungseinrichtung (50) so angeordnet und ausgebildet ist, dass Licht zur Bildaufrichtung nacheinander an der ersten reflektierenden Fläche (61) reflektiert wird, an einer dritten reflektierenden Fläche (63) reflektiert wird, durch die erste reflektierende Fläche (61) hindurchtritt, an einer vierten reflektierenden Fläche (64) reflektiert wird, durch die zweite reflektierende Fläche (62) hindurchtritt, an einer fünften reflektierenden Fläche (65) reflektiert wird, und an der zweiten reflektierenden Fläche (62) reflektiert wird.

3. Endoskop (10) nach dem vorangehenden Anspruch, bei dem die dritte reflektierende Fläche (63), die vierte reflektierende Fläche (64), und die fünfte reflektierende Fläche (65) **parallel zueinander** angeordnet sind.

4. Endoskop (10) nach einem der Ansprüche 2 oder 3, bei dem die dritte reflektierende Fläche (63), die vierte reflektierende Fläche (64), und die fünfte reflektierende Fläche (65) **parallel zur optischen Achse** (48) der Bildübertragungseinrichtung (40) angeordnet sind.

5. Endoskop (10) nach dem vorangehenden Anspruch, bei dem alle reflektierenden Flächen (61, 62, 63, 64, 65) der Bildaufrichtungseinrichtung (50), die an der Bildaufrichtung beteiligt sind, eben und **komplanar** sind.

6. Endoskop (10) nach nach einem der Ansprüche 2 bis 5, bei dem
an zwei einander gegenüberliegenden der sechs ebenen Oberflächenabschnitte des **Quaders** die Lichteintrittsfläche (67) der Bildaufrichtungseinrichtung (50) und die Lichtaustrittsfläche (69) der Bildaufrichtungseinrichtung (50) angeordnet sind,
an zwei weiteren einander gegenüberliegenden der sechs ebenen Oberflächenabschnitte des Quaders einerseits die vierte reflektierende Fläche (64) und andererseits die dritte und die fünfte reflektierende Fläche (63, 65) angeordnet sind.

7. Endoskop (10) nach einem der Ansprüche 2 bis 6, bei dem
die Bildaufrichtungseinrichtung (50) ein erstes Prisma (51), ein zweites Prisma (52) und ein drittes Prisma (53) umfasst,
das **erste Prisma** (51) eine Lichteintrittsfläche (67) der Bildaufrichtungseinrichtung (50), die erste reflektierende Fläche (61) und die dritte reflektierende Fläche (63) umfasst,
das **zweite Prisma** (52) die vierte reflektierende Fläche (64), eine Lichteintrittsfläche (54) gegenüber und parallel zu der ersten reflektierenden Fläche (61) und eine Lichtaustrittsfläche (55) gegenüber und parallel zu der zweiten reflektierenden Fläche (62) umfasst,
das **dritte Prisma** (53) eine Lichtaustrittsfläche (69) der Bildaufrichtungseinrichtung (50), die zweite reflektierende Fläche (62) und die fünfte reflektierende Fläche (65) umfasst.

8. Endoskop (10) nach einem der vorangehenden Ansprüche, bei dem die erste reflektierende Fläche (61) und die zweite reflektierende Fläche (62) der Bildaufrichtungseinrichtung (50) jeweils in einem Winkel von **30 Grad** zur optischen Achse (48) der Bildübertragungseinrichtung (40) angeordnet sind.

9. Endoskop (10) nach einem der vorangehenden Ansprüche, ferner mit
einer **weiteren reflektierenden Fläche** (32) am distalen Ende (11) des Endoskops (10), zum Einkoppeln von Licht, das von einem zu beobachtenden Objekt ausgeht, in die Bildübertragungseinrichtung (40), wobei die weitere reflektierende Fläche (32) zum Einstellen der Blickrichtung (12) um eine Schwenkachse (38) schwenkbar ist;
einer Einrichtung (60) zum **Koppeln der Bildaufrichtungseinrichtung** (50) **mit der weiteren reflektierenden Fläche** (32) derart, dass ein Schwenken der weiteren reflektierenden Fläche (32) um die Schwenkachse (38) mit einer Rotation der Bildaufrichtungseinrichtung (50) um eine Rotationsachse (58) parallel zur optischen Achse (48) der Bildübertragungseinrichtung (40) einher geht.

## Claims

1. **Endoscope** (10), comprising:
an **image transmission device** (40) in a shaft (20) for transmitting an image from the distal end (11) to the proximal end (12) of the endoscope (10);
an **image erecting device** (50) for erecting an image registered by the endoscope (10),
wherein the image erecting device (50) has a first **reflective surface** (61) **for angel-dependent reflection** and a second **reflective surface** (62) **for angle dependent reflection**,
wherein the image erecting device (50) is arranged and configured such that light for image erecting is successively reflected at the first reflective surface (61), passed through the first reflective surface (61) and the second reflective surface (62) and reflected at the second reflective surface (62),
**characterized in that**
the image erecting device (50) provides substantially the shape of a **cuboid**, wherein the cuboid provides a cross-section in a plane perpendicular to an optical axis of the image transmission device (40) that is at least substantially square shaped.

2. Endoscope (10) according to the preceding claim, wherein the image erecting device (50) is arranged and configured such that light for image erecting is successively reflected at the first reflective surface (61), reflected at a third reflective surface (63), passed through the first reflective surface (61), reflected at a fourth reflective surface (64), passed through the second reflective surface (62), reflected at a fifth reflective surface (65) and reflected at the second reflective surface (62).

3. Endoscope (10) according to the preceding claim, wherein the third reflective surface (63), the fourth reflective surface (64), and the fifth reflective surface (65) are arranged **parallel to one another.**

4. Endoscope (10) according to one of claims 2 and 3, wherein the third reflective surface (63), the fourth reflective surface (64), and the fifth reflective surface (65) are arranged **parallel to the optical axis** (48) of the image transmission device (40).

5. Endoscope (10) according to the preceding claim, wherein all reflective surfaces (61, 62, 63, 64, 65) of the image erecting device (50) which are part of the image erection are planar and **coplanar**.

6. Endoscope (10) according to one of the claims 2 through 5, wherein
the light-entry surface (67) of the image erecting device (50) and the light-exit surface (69) of the image erecting device (50) are arranged at two planar surface sections, lying opposite one another, of six planar surface sections of the image erecting device (50);
the fourth reflective surface (64), on the one hand, and the third and the fifth reflective surfaces (63, 65), on the other hand, are arranged at two further planar surface sections, lying opposite one another, of the six planar surface sections of the image erecting device.

7. Endoscope (10) according to one of the claims 2 through 6, wherein
the image erecting device (50) comprises a first prism (51), a second prism(52), and a third prism(53);
the **first prism** (51) comprises a light-entry surface (67) of the image erecting device (50), the first reflective surface (61), and the third reflective surface (63);
the **second prism** (52) comprises the fourth reflective surface (64), a light-entry surface (54) which is opposite and parallel to the first reflective surface (61), and a light-exit surface (55) which is opposite and parallel to the second reflective surface (62); and
the **third prism** (53) comprises a light-exit surface (69) of the image erecting device (50), the second reflective surface (62), and the fifth reflective surface (65).

8. Endoscope (10) according to one of the preceding claims, wherein the first reflective surface (61) and the second reflective surface (62) of the image erecting device (50) are each arranged at an angle of **30 degrees** with respect to the optical axis (48) of the image transmission device (40).

9. Endoscope (10) according to one of the preceding claims, further comprising
a **further reflective surface** (32) at the distal end (11) of the endoscope (10), for coupling light, emanating from an object to be observed, into the image transmission device (40), the further reflective surface (32) being pivotable about a pivot axis (38) for adjusting the viewing direction (12);
a device for **coupling the image erecting device** (50) **with the further reflective surface** (32) in such a way that pivoting the further reflective surface (32) about the pivot axis (38) is accompanied by a rotation of the image erecting device (50) about a rotational axis (58) parallel to the optical axis (48) of the image transmission device (40).

## Revendications

1. **Endoscope (10)**, comprenant :
un **dispositif de transmission d'image** (40) situé dans une tige (20) et destiné à transmettre une image de l'extrémité distale (11) à l'extrémité proximale (12) de l'endoscope (10) ;
un **dispositif de redressement d'image** (50) destiné à redresser une image acquise au moyen de l'endoscope (10),
le dispositif de redressement d'image (50) comportant une **première surface réfléchissante** (61) dont la réflexion dépend de l'angle, et une **deuxième surface réfléchissante** (62) dont la réflexion dépend de l'angle,
le dispositif de redressement d'image (50) étant disposé et conçu de sorte que, afin d'effectuer le redressement d'image, la lumière soit successivement réfléchie par la première surface réfléchissante (61), amenée à travers la première surface réfléchissante (61) et à travers la deuxième surface réfléchissante (62) et réfléchie par la deuxième surface réfléchissante (62),
**caractérisé en ce que**
le dispositif de redressement d'image (50) a sensiblement la forme d'un **parallélépipède,** le parallélépipède ayant une section transversale sensiblement carrée dans un plan perpendiculaire à l'axe optique du dispositif de transmission d'image (40).

2. Endoscope (10) selon la revendication précédente, dans lequel le dispositif de redressement d'image (50) est disposé et conçu de sorte que, pour effectuer le redressement d'image, la lumière soit successivement réfléchie par la première surface réfléchissante (61), réfléchie par une troisième surface réfléchissante (63), amenée à travers la première surface réfléchissante (61), réfléchie par une quatrième surface réfléchissante (64), amenée à travers la deuxième surface réfléchissante (62), réfléchie par une cinquième surface réfléchissante (65) et réfléchie par la deuxième surface réfléchissante (62).

3. Endoscope (10) selon la revendication précédente, dans lequel la troisième surface réfléchissante (63), la quatrième surface réfléchissante (64) et la cinquième surface réfléchissante (65) sont disposées **parallèlement les unes aux autres**.

4. Endoscope (10) selon l'une des revendications 2 et 3, dans lequel la troisième surface réfléchissante (63), la quatrième surface réfléchissante (64) et la cinquième surface réfléchissante (65) sont disposées **parallèlement à l'axe optique** (48) du dispositif de transmission d'image (40).

5. Endoscope (10) selon la revendication précédente, dans lequel toutes les surfaces réfléchissantes (61, 62, 63, 64, 65) du dispositif de redressement d'image (50), qui sont impliquées dans le redressement d'image, sont planes et **coplanaires.**

6. Endoscope (10) selon l'une des revendications 2 à 5, dans lequel
la surface d'entrée de lumière (67) du dispositif de redressement d'image (50) et la surface de sortie de lumière (69) du dispositif de redressement d'image (50) sont disposées sur deux des six portions de surface planes du **parallélépipède,** lesdites deux portions se faisant face,
la quatrième surface réfléchissante (64) d'une part et les troisième et cinquième surfaces réfléchissantes (63, 65) d'autre part sont disposées sur deux autres des six portions de surface planes du parallélépipède, lesdites deux autres portions se faisant face.

7. Endoscope (10) selon l'une des revendications 2 à 6, dans lequel
le dispositif de redressement d'image (50) comprend un premier prisme (51), un deuxième prisme (52) et un troisième prisme (53),
le **premier prisme** (51) comprend une surface d'entrée de lumière (67) du dispositif de redressement d'image (50), la première surface réfléchissante (61) et la troisième surface réfléchissante (63),
le **deuxième prisme** (52) comprend la quatrième surface réfléchissante (64), une surface d'entrée de lumière (54) opposée et parallèle à la première surface réfléchissante (61) et une surface de sortie de lumière (55) opposée et parallèle à la deuxième surface réfléchissante (62),
le **troisième prisme** (53) comprend une surface de sortie de lumière (69) du dispositif de redressement d'image (50), la deuxième surface réfléchissante (62) et la cinquième surface réfléchissante (65).

8. Endoscope (10) selon l'une des revendications précédentes, dans lequel la première surface réfléchissante (61) et la deuxième surface réfléchissante (62) du dispositif de redressement d'image (50) sont chacune disposées suivant un angle de **30 degrés** par rapport à l'axe optique (48) du dispositif de transmission d'image (40).

9. Endoscope (10) selon l'une des revendications précédentes, comprenant en outre
une **autre surface réfléchissante** (32) située à l'extrémité distale (11) de l'endoscope (10) et destinée à injecter par couplage la lumière, qui émane d'un objet à observer, dans le dispositif de transmission d'image (40), l'autre surface réfléchissante (32) pouvant pivoter sur un axe de pivotement (38) afin de régler la direction d'observation (12) ;
un dispositif (60) destiné à **coupler le dispositif de redressement d'image** (50) **à l'autre surface réfléchissante** (32) de manière à ce qu'un pivotement de l'autre surface réfléchissante (32) sur l'axe de pivotement (38) coïncide avec une rotation du dispositif de redressement d'image (50) sur un axe de rotation (58) parallèle à l'axe optique (48) du dispositif de transmission d'image (40).
